# EUROPEAN PATENT APPLICATION

(11) **EP 3 646 858 A1**
(43) Date of publication of application: **06.05.2020**
(21) Application number: 18822991.8
(22) Date of filing: 28.06.2018
(51) Int. Cl.: A61K 9/54, A61K 9/26

(54) **ENCAPSULATED FORMULATIONS**

(30) Priority: 30.06.2017 ES 201730871
(71) Applicant: Pharmalink, SL, 08290 Cerdanyola Del Valles (ES)
(72) Inventor: JANE AUBAREDA, Barbara, 08480 L'Ametlla Del Vallèa (ES)
(74) Representative: Juncosa Miró, Jaime
(86) International application number: PCT/ES2018/070465
(87) International publication number: WO 2019/002657

(57) **Abstract**

The invention relates to encapsulated formulations comprising a capsule comprising tablets, microgranules, powder, or granulated material with some or all of the following components or food supplements: plant extracts, vitamins, minerals, and amino acids. The capsule is used to produce a food supplement for the prevention and treatment of different types of disorders.

## Description

### Field of the Art

The present invention relates to food supplements in the form of capsules comprising different formulations of different components with different release forms, as well as to the use thereof to counteract different disorders.

### Background of the Invention

A food supplement is a vitamin, mineral, plant extract, or amino acid that is taken to improve health or wellbeing.

Although most of these supplements can be obtained with a varied diet through foods, there are many cases in which supplements are necessary, such as for example, among vegetarians, pregnant women, breastfeeding women, women with heavy menstrual periods, menopausal and postmenopausal women, people with gastrointestinal diseases, such as lactose intolerance or allergy to certain foods, people with stomach, liver, pancreatic, or gall bladder diseases, and also for the purpose of reducing the risk of cardiovascular diseases, cancer, etc.

According to the U.S. Department of Agriculture, most adults fail to obtain a sufficient amount of the following nutrients from their regular diet:

| Nutrient | Recommended amount |
|---|---|
| Calcium | 1000 mg |
| Potassium | 4700 mg |
| Fiber | 25 g (women) |
| | 38 g (men) |
| Magnesium | 320 g (women) |
| | 420 g (men) |
| Vitamin A | 2310 IU (international units) for women |
| | 3000 IU for men |
| Vitamin C | 75 mg for women |
| | 90 mg for men |
| Vitamin E | 15 mg |

Deficiency of some nutrients has been correlated with a higher incidence of certain diseases, as well as disorders of different severity such as sleep disorders, anxiety, joint pain, migraines.

The human body utilizes these nutrients for many different functions, including bone formation, hormone production, and heart rate regulation. Many people with mild nutrient deficiencies are predisposed to develop age-related diseases, such as cancer, heart diseases, and loss of immune function or brain functions.

Different activities have been described for different food supplements.

Melatonin is produced in the body stimulated by the absence of light, causing the concentration of this hormone in the body to increase at nightfall, with a maximum peak between 2 am and 4 am. External supply of this hormone favors sleep onset. *("Scientific Opinion on the substantiation of a health claim related to melatonin and reduction of sleep onset latency" (ID 1698, 1780, 4080) pursuant to Article 13 of Regulation (EC) No 1924*/*2006 and "*Aging and Circadian Rhythms". Sleep Med Clin. 2015 December; 10(4): 423-434. doi:10.1016/j.jsmc.2015.08.002*).*

GABA is an inhibitory neurotransmitter in the mature brain. It is thought that GABA may influence the catabolism of serotonin to N-acetylserotonin and melatonin. In that sense, the concomitant use of GABA and melatonin has a synergistic action *("*Neurotransmitters as food supplements: the effects of GABA on brain and behaviour" Evert Boonstra, Roy de Kleijn, Lorenza S. Colzato, Anneke Alkemade, Birte U. Forstmann and Sander Nieuwenhuis, Cognitive Psychology Unit, Institute of Psychology, Leiden University, Leid*en).*

The *Passiflora* extract is widely used for treating cases of nervosism and insomnia or for relieving anxiety. This is due to the sedative properties of the plant. Research that has been conducted demonstrates that the compounds in the plant stimulate an increased production of brain gamma aminobutyric acid or GABA, producing a mild sedative effect *("Efficacy and safety of a polyherbal sedative-hypnotic formulation NSF-3 in primary insomnia in comparison to zolpidem: A randomized controlled trial" Niteeka Maroo, Avijit Hazra, and Tapas Das).*

*Valeriana* extract, known since time immemorial as valerian, is used as an anxiolytic. At present, its efficacy has been proven in different clinical studies *("Efficacy and safety of a polyherbal sedative-hypnotic formulation NSF-3 in primary insomnia in comparison to zolpidem: A randomized controlled trial" Niteeka Maroo, Avijit Hazra, and Tapas Das).*

Kawa-kawa, kava kava, or kava (*Piper methysticum*) is a plant of Polynesian origin. The roots of the plant are used to produce a drink with sedative and anesthetic properties. Its active ingredients are called kavalactones. (*Kavain, the Major Constituent of the Anxiolytic Kava Extract, Potentiates GABAA Receptors: Functional Characteristics and Molecular Mechanism Han Chow Chua, Emillie T. H. Christensen, Kirsten Hoestgaard-Jensen, Leonny Y. Hartiadi, Iqbal Ramzan, Anders A. Jensen, Nathan L. Absalom, Mary Chebib).*

It is postulated that the mechanism of the anxiolytic activity of kava is due to an improved binding to GABA receptors type A *(Kavain, the Major Constituent of the Anxiolytic Kava Extract, Potentiates GABAA Receptors: Functional Characteristics and Molecular Mechanism Han Chow Chua, Emillie T. H. Christensen, Kirsten Hoestgaard-Jensen, Leonny Y. Hartiadi, Iqbal Ramzan, Anders A. Jensen, Nathan L. Absalom, Mary Chebib).*

Another food supplement is saffron (*Crocus sativus L.*), the main bioactive components of which are crocins and safranal which impart the characteristic color and aroma to saffron. Crocins are dopamine and norepinephrine uptake inhibitors, whereas safranal acts mainly on serotonin uptake. The antioxidant properties of saffron derivatives may also be relevant. Mood disorders are associated with high oxidative stress and a lack of exogenous antioxidants, affecting immune and inflammatory responses such that it can promote neurodegeneration. There is suitable evidence that antioxidants in saffron extracts protect against oxidative stress in the central nervous system, constituting a second potential mechanism of therapeutic action. (Kell Graham, Rao Amanda, Beccaria Gavin, Clayton Paul, Inarejos-Garcia Antonio Manuel, Prodanov Marin. affron® a novel saffron extract (Crocus sativus L.) improves mood and sleep quality in healthy adults over 4 weeks in a double-blind, parallel, randomized, placebo-controlled clinical trial. Complementary Therapies in Medicine http://dx.doi.org/10.1016/j.ctim.2017.06.001*.)*

Since late 1800, *Echinacea* plant species, mainly *Echinacea angustifolia*, constituted one of the most widely used medicinal plants for fighting upper respiratory tract infections and for curing wounds. In the 20^{th} century, with the discovery of antibiotics, interest in the medicinal use of *Echinacea* faded away. The use of *Echinacea* became popular again mainly due to the emergence of bacterial resistances to antibiotics. There are 9 *Echinacea* species, three of which are cultivated and well known for their phytotherapeutic properties: *Echinacea purpurea*, *Echinacea pallida*, and *Echinacea angustifolia.* The root of *Echinacea* is made up of phenolic compounds derived from caffeic acids such as echinacoside. As indicated in the monograph of the European Medicines Agency (EMA), the root of *Echinacea* stimulates the immune system. According to the EMA and various scientific studies that have been conducted, the root of *Echinacea* provides immunomodulating activity: *Echinacea* facilitates the action of immune cells and makes them more effective against pathogenic microorganisms. The plant increases the number and activity of immune system cells, particularly white blood cells and lymphocytes. It stimulates the action of macrophages *(*Luettig B, Steinmuller C, Gifford GE, Wagner H, Lohmann-Matthes ML. Macrophage activation by the polysaccharide arabinogalactan isolated from plant cell cultures of Echinacea purpurea. J Natl Cancer Inst. 1989 May 3; 81 (9): 669-75*. PMID 2785214)* and induces their cytotoxicity *(*Stimpel M, Proksch A, Wagner H, Lohmann-Matthes ML. Macrophage activation and induction of macrophage cytotoxicity by purified polysaccharide fractions from the plant Echinacea purpurea. Infect Immun. 1984 Dec;46 (3): 845-9*. PMID 6389368).* It allows the formation of cytokines, cells responsible for the immune response.

Magnesium is a mineral that acts on the neurological system, favoring sleep and relaxation, although the mechanism of action is little known. Situations of stress have been associated with a greater loss of magnesium in the body. (The effects of magnesium supplementation on subjective anxiety Neil Bernard Boyle, Clare L. Lawton, Louise Dye School of Psychology, University of Leeds, Leeds, LS2 9JT, UK*).* Magnesium is typically present as an Mg²⁺ ion in all the cells of the human body. The biologically active form of ATP, the main form of energy on the cellular level, is linked to magnesium. A recent meta-analysis concludes that the lack of Mg²⁺ is strongly associated with migraine attacks.

Vitamin B6 intervenes in the metabolism of mood-regulating neurotransmitters, such as serotonin, and in the synthesis of dopamine, adrenaline, norepinephrine, and GABA (gamma aminobutyric acid), so it aids in improving mood in cases of depression, stress, and sleep disturbances associated with a lack of serotonin. There are studies demonstrating that vitamin B6 improves the anxiolytic effect when administered together with magnesium. *("*The effects of magnesium supplementation on subjective anxiety" Neil Bernard Boyle, Clare L. Lawton, Louise Dye School of Psychology, University of Leeds, Leeds, LS2 9JT, UK*.;* De Souza MC, Walker AF, Robinson PA, Bolland K. "A synergistic effect of a daily supplement for 1 month of 200 mg magnesium plus 50 mg vitamin B6 for the relief of anxiety-related premenstrual symptoms: a randomized, double-blind, crossover study". J Womens Health Gend Based Med 2000; 9: 131-9*; "*Magnesium deficiency induces anxiety and HPA axis dysregulation: Modulation by therapeutic drug treatment" S.B. Sartori, N. Whittle, A. Hetzenauer, N. Singewald*).*

5-hydroxytryptophan (5-HTP) is a natural amino acid and a precursor and intermediate chemical compound in the biosynthesis of serotonin and melatonin neurotransmitters from tryptophan. *("*5-Hydroxytryptophan: A Clinically-effective Serotonin Precursor" by Timothy C. Birdsall, N.D., Tryptophan overloading activates brain regions involved with cognition, mood and anxiety Luana C.A. Silva, Milena B. Viana, José S. Andrade, Melyssa A. Souza, Isabel C. Céspedes and Vânia D'Almeida*).*

Coenzyme Q10 is found mainly in the mitochondria where energy is generated in the form of ATP by means of the Krebs cycle in the electron transport chain and participates in aerobic cellular respiration. Ninety-five percent of the energy of the human body is generated in this manner. It is believed that migraines are a mitochondrial disorder and mitochondrial dysfunction is improved with coenzyme Q10, so a supplementation with CoQ10 may have a beneficial effect for the prophylaxis of migraine attacks. *("Improvement of migraine symptoms with a proprietary supplement containing riboflavin, magnesium and Q10: a randomized, placebo-controlled, double-blind, multicenter trial" Charly Gaul, Hans-Christoph Diener, Ulrich Danesch and on behalf of the Migravent*® *Study Group).*

Vitamin B2 (riboflavin) is a group B vitamin involved in mitochondrial ATP synthesis. The recommended daily doses are 1.7 mg for adults. Endogenous riboflavin assimilation occurs fundamentally in the small intestine with a plasma half-life of 1 to 2 hours. Amounts greater than 30 - 50 mg of B2 are not assimilated by the body, with the excess being excreted in urine. *("Improvement of migraine symptoms with a proprietary supplement containing riboflavin, magnesium and Q10: a randomized, placebo-controlled, double-blind, multicenter trial" Charly Gaul, Hans-Christoph Diener, Ulrich Danesch and on behalf of the Migravent*® *Study Group; "Effectiveness of Vitamin B2 versus Sodium Valproate in Migraine Prophylaxis: a randomized clinical trial".* Abolghasem Rahimdel, Ahmad Zeinali, Pouria Yazdian-anari, Rahele Hajizadeh, Ehsan Arefnia; "Intestinal absorption of water-soluble vitamins in health and disease" Hamid M. Said School of Medicine, University of California-Irvine, Irvine, CA 92697, U.S.A*., and Department of Medicine, VA Medical Center, Long Beach, CA 90822, U.S.A.).*

Vitamin D receptor (VDR) and vitamin D activate the 1-α-hydroxylase enzyme (CYP27B1) which is expressed in many cell types not involved in bone and mineral metabolism, such as the intestine, pancreas, prostate, and immune system cells *(Holick, M.F. Vitamin D deficiency.* N. Engl. J. Med. 2007, 357, 266-281*.* Battault, S.; Whiting, S.J.; Peltier, S.L.; Sadrin, S.; Gerber, G.; Maixent, J.M. Vitamin D metabolism, functions and needs: From science to health claims. Eur. J. Nutr. 2013, 52, 429-441*).* This suggests a significant impact of vitamin D in the field of human immunology, i.e., the extrarenal synthesis of the calcitriol metabolite active substance -1,25(OH)₂D-, by immune cells and peripheral tissues. In June 2015, the European Food Safety Authority (EFSA) concluded that there was a cause-effect relationship between vitamin D intake in the diet and normal functioning of the immune system *(doi:10.2903*/*j.efsa.2015.4182).* However, the long-term effects of high doses of vitamin D supplements are little known *[*A. Zittermann, J. B. Ernst, S. Prokop et al., "Effect of vitamin D on all-cause mortality in heart failure (EVITA): a 3-year randomized clinical trial with 4000 IU vitamin D daily," European Heart Journal, vol. 38, no. 29, pp. 2279-2286, 2017*].* Vitamin D induces the endogenous production of vitamin K-dependent proteins which require vitamin K for carboxylation and proper functioning.

Vitamin K is a fat-soluble vitamin like vitamin D that exists in two forms: K1 (phylloquinone, found mainly in long leaf plants) and K2 (menaquinone, present mainly in milk fermented and secreted by lactic ferments in the intestine) *[*S. L. Booth and A. RajabiAl, "Determinants of vitamin K status in humans," Vitamins and Hormones, vol. 78, pp. 1-22, 2008*.].* Vitamin K1 is transported mainly to the liver, regulating coagulation factor production, whereas vitamin K2 is transported to the bone and vascular wall, regulating the activity of the matrix Gla protein (MGP) and osteocalcin (bone Gla protein) - the main vitamin K-dependent proteins. These proteins require protein K for carboxylation and proper functioning. When plasma levels of vitamin K are insufficient, a greater proportion of MGP and osteocalcin remains decarboxylated, which is associated with a higher risk of cardiovascular disease *[*S. A. Lanham-New, "Importance of calcium, vitamin D and vitamin K for osteoporosis prevention and treatment," The Proceedings of the Nutrition Society, vol. 67, pages 163-176, 2008*.].*

Chondroitin sulfate (CS) is a main component of the extracellular matrix (ECM) of many tissues, including cartilage, bone, skin, ligaments, and tendons. Osteoarthritis (OA) is characterized by progressive change in the structure and metabolism of joint tissues, mainly degradation, subchondral bone sclerosis, and inflammation of the synovial membrane. CS is a sulfated glycosaminoglycan (GAG) made up of a long, unbranched polysaccharide chain with repeating N-acetylgalactosamine and glucuronic acid disaccharide structure. CS is responsible for many of the important properties of cartilage, such as strength and elasticity. Its high aggrecan content plays an important role by allowing cartilage to withstand pressure under several load conditions. The sulfation profile of chondroitin has been described in cartilage [Mourao, 1988]. Changes in the CS structure have been described in tissues with OA [Caterson *et al.* 1990] and a reduced CS-6: CS-4 has been found in the synovial fluid and cartilage of patients with OA [Shinmei *et al.* 1992]. For more than three decades, [Schwartz and Dorfman, 1975] proposed that CS, as therapy for damaged cartilage, could provide the basic elements for the synthesis of the new component of the matrix (ECM), the administration of CS acting in favor of matrix regeneration.

Glucosamine is one of the main macromolecules constituting the extracellular matrix (ECM) such as glycosaminoglycans. The relevant mechanisms of action of glucosamine include the anti-inflammatory effect, pro-anabolic effect, promotion of osteoblast proliferation, and catabolic intermediate inhibition. Chan *et al.* reported that glucosamine plus chondroitin showed complementary anti-catabolic and anti-inflammatory effects in comparison with the use of glucosamine or chondroitin alone.

Osteoarthritis (OA) mainly affects joints such as fingers, knees, hip, backbone, wrists, elbows, shoulders, and ankles. Knee OA is the most common. There can be found inside the knee a substance called synovial fluid which acts as a damper for dampening the weight of the body when walking, jumping, and running, while at the same time lubricating the joints, allowing a smooth, friction-free movement thereof. In a knee with OA, the synovial fluid suffered changes and lost these dampening and lubricating properties, leaving the cartilage and soft tissues unprotected, which causes joint pain and stiff joint, until reaching advanced stages, i.e., cartilage destruction and tendon and bone damage.

Joint inflammation and pain are the main symptoms of this disease, where it may become disabling for those suffering from said disease. Early treatment can help to delay the progression of the disease and reduce its symptoms.

*Curcuma longa* (turmeric) has a long history of being used in ayurvedic medicine as treatment for inflammatory diseases. Turmeric is made up mainly of three curcuminoids: curcumin (diferuloylmethane, the primary constituent responsible for its intense yellow color), dimethoxycurcumin, and bisdemethoxycurcumin. Turmeric interacts with different biomolecules involved in inflammatory processes, and various studies have been published demonstrating the anti-inflammatory capacity of turmeric. However, due to the metabolization speed of curcumin and its limited bioavailability, it was necessary to associate the intake of this molecule with other compounds intended for enhancing the bioavailability of this molecule, among others the 10:1 association with piperine to enhance the anti-inflammatory efficacy thereof ("Anti-inflammatory Properties of Curcumin, a Major Constituent of Curcuma longa: A Review of Preclinical and Clinical Research". Julie S. Jurenka, MT (ASCP)).

The association of curcumin with the alkaloid piperine, a constituent of black pepper and long pepper (*Piper nigrum* and *Piper longum*, respectively), increases the bioavailability of curcumin by 20-fold as a result of the inhibition of hepatic and intestinal glucuronidation.

Eggshell membrane, i.e., the film which separates from the shell typically after boiling or scalding an egg, is what remains of the different membranes protecting the embryo during its development. Supplementing the diet with eggshell membrane has demonstrated good results in joint diseases. Its main composition is collagen, mostly type I collagen, among other metabolites which have proven action on joint problems such as glycosaminoglycans and proteins responsible for eggshell mineralization. Furthermore, eggshell membrane is very rich in sulfur amino acids (SAAs) which contribute to joint inflammation reduction, such as dermatan sulfate and chondroitin sulfate and sulfated glycoproteins including hexosamines, such as glucosamine. Other components identified in eggshell membranes are hyaluronic acid, sialic acid, desmosine and isodesmosine, ovotransferrin, lysyl oxidase, lysozyme, and β-N-acetylglucosaminidase. The discovery of eggshell membrane as a source of collagen combined with glucosamine, chondroitin, and hyaluronic acid associated with a variety of other trace elements with synergistic anti-inflammatory and reparative collagen action makes this compound an ideal candidate in diets for people with joint pain.

Resveratrol is a polyphenol that occurs naturally in grapes and red wine and has been demonstrated to exert a significant antioxidant effect. Since it was first isolated in the mid-twentieth century, many scientific studies and articles have been published with respect to this molecule, highlighting its antioxidant and anti-cancer properties and its relationship with a delayed aging process.

Rosehip or rosa mosqueta is the pome fruit of shrubs of the genus *Rosa.* Rosehip can be eaten raw, being an excellent source of antioxidants among which vitamin C, vitamins A, D, E, and antioxidant flavonoids stand out.

Acai is the fleshy fruit of the acai palm, common in the vegetation of the Amazon rainforest. Its consumption has become popular recently as a result of its great antioxidant power exceeding other fruits such as blueberries or raspberries.

Quercetin is a flavonol with a significant antioxidant activity that is present in fruits and vegetables, particularly in onions, apples, grapes, broccolis, or tea. In these foods, quercetin is linked to polysaccharides, forming hydrophilic glucosides with a low bioavailability. The body assimilates it following hydrolysis in the small intestine. For this reason, the formulation of the present invention keeps the active ingredient in the stomach to be rapidly released once gastric emptying has taken place and the active ingredient reaches the upper part of the small intestine.

Some untreatable eye diseases and injuries result from continuous exposure to light, together with the highly oxygenated environment existing inside the eyeball. Two of the main causes of visual impairment and blindness are age-related macular degeneration (ARMD) and cataracts. Macular degeneration is a progressive disorder affecting the central part of the retina, called macula. Macular degeneration causes gradual loss of photoreceptor cells and is the main cause of irreversible blindness. Prevention is crucial since successful treatments for this disease are few and far between. Both cataracts and ARMD seem to be related to light-induced oxidative processes inside the eye. While cataracts affect the eye lens (crystalline lens), the macula is damaged in ARMD. Both the crystalline lens and the retina are continuously exposed to light (particularly blue light) and oxygen, producing free radicals. In the formation of cataracts, free radicals seem to alter the crystalline proteins of the eye lens, causing them to cluster together, and also damage the proteolytic enzymes that would normally remove the damaged proteins at night. One of the challenges associated with ocular nutrition is the limited capacity of the body to deliver nutrients to the suitable areas in the eye. It has been demonstrated that a higher intake of carotenoids in the diet is associated with a lower risk of ARMD. Among the carotenoids, scientific studies have proven the importance of two carotenoids in ocular health: lutein and zeaxanthin. Lutein and zeaxanthin are oxygenated carotenoids called xanthophylls. Since it is oxygenated, a xanthophyll has greater antioxidant capacity than many other carotenoids. Among the many carotenoids in the diet, the human retina selectively accumulates only two: zeaxanthin and lutein. Their concentration is so high in the macula and in the crystalline lens that carotenoids are visible as a dark yellow spot called macular pigment. The biophysical and biochemical capacities of zeaxanthin and lutein can play a role in the pathogenesis and progression of retinal diseases. For example, lutein and zeaxanthin is capable of absorbing blue light before it reaches the photoreceptors *(*SanGiovanni, J.P.; Neuringer, M. The putative role of lutein and zeaxanthin as protective agents against age-related macular degeneration: Promise of molecular genetics for guiding mechanistic and translational research in the field. Am. J. Clin. Nutr. 2012, 96, 10*.).* Another carotenoid which has been associated with ocular health, but which has not received as much attention as lutein and zeaxanthin, is astaxanthin. Astaxanthin is a natural red carotenoid pigment. This xanthophyll is a fat-soluble nutrient, which is a potential antioxidant that is more powerful than other antioxidants such as beta-carotene, vitamin E, and vitamin C. Astaxanthin has demonstrated a powerful antioxidant activity and other properties that are beneficial for human health. *(*O'Connor I, O'Brien N. Modulation of UVA light-induced oxidative stress by beta-carotene, lutein and astaxanthin in cultured fibroblasts. J Dermatol Sci 1998;16: 226-30*);* Piermarocchi S, Saviano S, Parisi V, Tedeschi M, Panozzo G, Scarpa G, Boschi G, Lo Giudice G; Carmis Study Group. Carotenoids in Age-related Maculopathy Italian Study (CARMIS): two-year results of a randomized study. Eur J Ophthalmol. 2012 Mar-Apr;22(2):216-25. doi: 10.5301/ejo.5000069*. PubMed PMID: 22009916.)*

Zinc and copper are trace elements concentrated in photoreceptors and the retinal pigment epithelium (RPE) of the human eye (Galin MA, Nano HD, Hall T. Ocular zinc concentration. Invest Ophthalmol 1962;1:142-148*).* Zinc and copper work as cofactors in several ocular enzymes, including copper zinc dismutase superoxide, a part of the primary antioxidant system used for regulating oxidative stress.

All these food supplements are added to the nutritional diets of people suffering from different types of disorders, such as sleep disorders, anxiety, joint pain, migraines, and others, in different dosage forms, with different administration regimens.

This presents the problem that the patient must be able to follow an organized dosage regimen of different forms at different times of the day, before, after, or with food, which the patient may find difficult to follow, as well as a lower efficacy of said supplements. Dosing complexity affects compliance with the prescription.

Wald and Law (Br. Med. J. 326, No. 7404, 1419-23, 2003) defined the term "polypill," referring to the combination of medicinal products such as statins, antihypertensives, aspirin, and vitamins, such as folic acid, for use in a single daily dose. These authors recommend the use of the polypill as a way to achieve a significant effect in cardiovascular disease prevention with minimal adverse effects. The strategy of the polypill containing several effective components for reducing cardiovascular risk factors would thereby prevent a high incidence of heart attacks and strokes. They concluded that the polypill strategy would be safe and that its widespread use would have a greater impact on cardiovascular disease prevention in western countries than any other intervention, and they estimated that the polypill could reduce the incidence of coronary diseases and ictus by up to 88 and 80%, respectively.

Patent EP 2273985 A1 of Ferrer Internacional offers new compositions in the form of capsules comprising a variable number of acetylsalicylic acid tablets, a variable number of tablets of an HMG-CoA reductase inhibitor and a variable number of tablets of an ACE inhibitor, such that the number of tablets of each active ingredient can be customized to the characteristics of the individual patients or subgroups of the population they are intended for.

However, the preparation of "polypills" of food supplements for the treatment of different types of disorders, such as sleep disorders, anxiety, joint pain, or migraines, has not been described up until now.

### Disclosure of the Invention

The present invention aims to solve the problems involved in the administration, to one and the same patient, of different food supplements that act on different points of the body, with different mechanisms and different absorption rates, and may have problems interacting with one another, as it consists of a unit dosage form, i.e., a capsule, containing therein different dosage forms of the different food supplements to be administered (tablets, microgranules, powder, granulated material), in different release forms, such that the administration of each and every one of the food supplements required for treating a specific disorder is achieved in a single capsule with the dose and the administration regimen required to achieve the desired amounts of each of the supplements.

In this sense, the object of the present invention is to provide new compositions in the form of capsules comprising a variable number of tablets, microgranules, powder, or granulated material with different food supplements, such as: plant extracts, vitamins, minerals, and/or amino acids.

The capsules of the present invention can be of plant or animal origin, preferably the capsules are capsules no. 0 of plant origin, and particularly transparent hypromellose capsules no. 0 of plant origin (VCAPS® Plus natura).

The food supplements used in the present invention are formulated in unit doses in the form of tablets, microgranules, powder, or granulated material, the release of which may or may not be modified.

The individual doses contained in the capsule can range between 0 and 6 tablets with one or more of components a) to d) and microgranules, powder, or granulated material with the rest of the components, preferably
a) between 0 and 3 tablets with plant extracts,
b) between 0 and 3 tablets with vitamins,
c) between 0 and 3 tablets with minerals, and
d) between 0 and 3 tablets with amino acids

According to the present invention, the plant extracts used are selected from: coenzyme Q10, *Hypericum*, *Curcuma kwangsiensis*, *Curcuma phaeocaulis*, *Curcuma zedoaria*, *Curcuma wenyujin*, *Curcuma aromatica*, *Echinacea* (*Equinacea angustifolia*) extract, *Harpagophytum procumbens*, *Harpagophytum zeyheri*, Mimosaceae (*Piptadeniastrum africanum*), *Passiflora* (*Passiflora caerulea*, *Passiflora edulis*, *Passiflora decaisneana*, *Passiflora manicata*) extract, *Valeriana (Valeriana collina, Valeriana pratensis, Valeriana repens, Valeriana sambucifolia*) extract, kava-kava (amount of extract equivalent to 100 mg of kavalactones), *Griffonia physocarpa*, *Griffonia speciosa*, *Griffonia tessmannii*, *Matricaria perforata*, *Matricaria recutita*, *Matricaria discoidea*, *Matricaria aurea*, *Tanacetum corymbosum*, *Tanacetum microphyllum*, *Chamaemelum nobile*, *Helichrysum italicum*, *Santolina chamaecyparissus*, saffron, L-5-hydroxytryptophan (5-HTP) extract, carotenoids (astaxanthin, lutein, zeaxanthin), preferably 5-HTP, dry seed extract of *Griffonia simplicifolia*, *Tanacetum parthenium*, *Passiflora incarnata*, *Valeriana officinalis,* saffron, *Echinacea angustifolia*, astaxanthin, lutein, and zeaxanthin.

According to the present invention, the vitamins used are selected from vitamins: A (retinol), B1 (thiamine), B2 (riboflavin), B3 (niacin), B5 (pantothenic acid), B6 (pyridoxine), B8 (biotin), B9 (folic acid), B12 (cobalamin), C (ascorbic acid), D (calciferol), E (α-tocopherol), K (phylloquinone), preferably vitamin B6 (pyridoxine), B2 (riboflavin), C (ascorbic acid), D, and K2 that is coated.

According to the present invention, the minerals used are selected from magnesium, calcium, phosphorus, sodium, potassium, chloride, iron, zinc, iodine, copper, manganese, fluorine, chromium, selenium, molybdenum, preferably magnesium oxide, iron, copper, zinc, and magnesium chloride.

According to the present invention, the amino acids used are selected from tryptophan, valine, histidine, arginine, methionine, leucine, isoleucine, lysine, phenylalanine, threonine, glycine, proline, serine, asparagine, preferably L-5-hydroxytryptophan (5-HTP).

According to the present invention, the unit dosage forms contained in the capsule are tablets, microgranules, powder, or granulated material with any of components a) to d), which can furthermore contain excipients of the type: diluent, binder, disintegrant, lubricant, surfactant, sweetener, dye, preservative, stabilizer, flavor enhancer, pH regulator, coatings.

According to the present invention, the diluents used in the unit dosage forms contained in the capsules comprise hydroxypropyl methylcellulose, calcium phosphate, lactose, dextrose, sugar, starch, modified starch, corn starch, mannitol, sorbitol, inorganic salts, microcrystalline cellulose, cellulose, calcium sulfate, calcium carbonate, xylitol, lactitol.

According to the present invention, the tablets and/or microgranules can be formed by an inert lipophilic or hydrophilic matrix which modifies the solubilization of the different ingredients making up same to provide different release characteristics.

According to the present invention, the tablets and/or microgranules can be coated with a protective coating, enteric coating, dye, and/or film to provide different release characteristics.

A particular object of the present invention is the development of formulations in capsules for the treatment of sleep disorders, containing tablets with melatonin which allow the precise release thereof, supplying the suitable amount when the body is in need thereof, thereby enhancing the effectiveness of insomnia treatment.

Up until now, sleep disorders have been treated with different nutritional supplements that act on different points of the body, with different mechanisms, different absorption rates, and different administration regimens.

Therefore, an object of the present invention is the combined use of melatonin, GABA, *Valeriana* extract, and *Passiflora* extract, which has shown a synergistic effect useful in promoting relaxation and calmness to first facilitate sleep onset and then maintaining same as a result of the special controlled-release formulation of melatonin and GABA.

Another object of the present invention is a formulation in the form of a capsule containing a controlled-release tablet with vitamin B6 and a controlled-release tablet with 5-HTP, which allows making vitamin B6 and 5-HTP gradually available to the body, facilitating prolonged serotonin stimulation. The same capsule contains two tablets with magnesium enhancing the anxiolytic effect of vitamin B6.

An object of the present invention is the use of a capsule containing a controlled-release tablet with vitamin B6, a controlled-release tablet with 5-HTP, and two tablets with magnesium for the treatment of anxiety.

Another object of the present invention is a formulation in the form of a capsule containing a controlled-release tablet with vitamin B6 and a controlled-release tablet with 5-HTP and saffron extract, which allows making vitamin B6 and 5-HTP gradually available to the body, facilitating prolonged serotonin stimulation. The same capsule contains two tablets with magnesium enhancing the anxiolytic effect of vitamin B6.

An object of the present invention is the use of a capsule containing a controlled-release tablet with vitamin B6, a controlled-release tablet combining saffron and 5-HTP, and two tablets with magnesium for the treatment of anxiety.

Another object of the invention is twice-a-day (day and night) formulations for the treatment of joint pain with mainly a regenerative function for the night and a formulation with anti-inflammatory action for the day.

The capsule to be administered at night contains chondroitin sulfate and glucosamine to beneficially contribute to the regeneration of damaged joint tissue. It also contains vitamin C, which is a cofactor for different enzymes intervening in the biosynthesis of collagen, the structural component of various body tissues such as bones, cartilage, gums, skin, tendons, and blood vessels, and curcumin with an anti-inflammatory effect. The capsule to be administered during the day contains two tablets with eggshell membrane: one being an intermediate-release tablet and another being a controlled-release tablet for improving absorption and reducing transport system saturation, and a controlled-release tablet with curcumin plus piperine: controlled release over 12 hours allows improving curcumin assimilation, thereby helping to reduce inflammation caused in the joints during the day.

Another object of the invention is formulations in capsules containing tablets with CoQ10, vitamin B2, and magnesium for the treatment of migraines.

Another object of the present invention is a formulation in the form of a capsule containing a controlled-release tablet with vitamin D plus K2 and a controlled-release tablet with iron plus vitamin C, which allows making Vitamin D and iron gradually available to the body, providing vitamin K2 to aid in the proper physiological function of vitamin D and vitamin C supplementation so as to improve iron absorption in the small intestine, facilitating the prolonged absorption thereof. The same capsule contains two tablets with *Echinacea* enhancing the effect of vitamin D and iron on the immune system.

An object of the present invention is the use of a capsule containing a controlled-release tablet with vitamin D3/K2, a controlled-release tablet with iron plus vitamin C, and two tablets with *Echinacea* extract for normal functioning of the immune system.

Another object of the present invention is a formulation in the form of a capsule containing a controlled-release tablet with astaxanthin which allows making astaxanthin gradually available to the body, helping to protect the eye from daytime oxidative stress. The same capsule contains two tablets with lutein and zeaxanthin enhancing the effect on ocular protection. The capsule is completed with a gastro-resistant tablet containing zinc and copper to assure the required supply of these two metals for proper eyesight functioning.

An object of the present invention is the use of a capsule containing a controlled-release tablet with astaxanthin, a gastro-resistant tablet with zinc and copper, and two tablets with lutein and zeaxanthin for protecting the eye from wear caused by oxidative stress and prolonged exposure to blue light from electronic apparatus displays.

The different formulations can have different release rates. A controlled-release formulation assures its prolonged effect without requiring several intakes a day.

### Detailed Description of the Invention

The capsules of the present invention comprise separate dosage forms of different food supplements. The preparation of different unit dosage forms of each of the food supplements allows providing a different release rate for each of them while at same time reducing the interaction between them and between the different pharmaceutically acceptable excipients of each of the forms.

At the same time, the complicated processes which combine different known active ingredients of the state of the art into a single unit dosage form using, for example, bilayer or trilayer tablets with or without intermediate barrier layers, are overcome.

In general, multiple dosage forms are more advantageous than unit dosage forms because they are dispersed in the gastrointestinal tract in a homogenous manner and transmitted from the stomach to the intestines. The effective dispersion of the active agents in the stomach is assured and bioavailability is thus improved. Multiple dosage forms also have the advantage of being able to be formulated such that they allow different release characteristics of the different active agents and/or natural extracts.

Therefore, the capsules of the present invention comprise a variable number of tablets, microgranules, powder, or granulated material with different food supplements, such as: plant extracts, vitamins, minerals and/or amino acids.

The unit dosage forms can be coated with a protective coating, enteric coating, film, and/or coatings which can provide different release rates.

The formulation of vitamins object of the present invention has been designed as a tablet with a gastro-resistant coating to assure that the vitamin is released once gastric emptying to the small intestine has taken place, likewise assuring maximum bioavailability by means of a delayed formulation which gradually releases the vitamin. Said tablet likewise incorporates a certain amount of citric acid for improving the stability of riboflavin which is unstable at an alkaline pH and protected from light due to its opaque coating to prevent photodegradation.

The excipients preferably used in the present invention are: a) microcrystalline cellulose, dicalcium phosphate anhydrous or hydrate, or a combination of both as a loading vehicle, b) colloidal silica as a glidant and stearic acid, sodium stearyl fumarate, talc, magnesium stearate, and/or glyceryl palmitostearate as lubricants, c) excipients such as hydroxypropyl methylcellulose (HPMC), hydroxyethyl cellulose (HEC), hydroxypropyl cellulose (HPC), and sodium carboxymethyl cellulose (Na CMC) as agents for modifying the release of the active ingredient present in the formulation, and d) calcium alginate or xanthan gum or lipophilic substances such as hydrogenated vegetable oil as gelling agents, preferably hydroxypropyl methylcellulose (HPMC) with a degree of viscosity between 11000 and 30000 mPa·s, preferably between 3000 and 5600 mPa·s.

The conventional techniques used in the pharmaceutical technology for the preparation of different unit dosage forms: tablets, microgranules, powder, or granulated material, which are described by way of examples below were used.

### Tablets

The different tablets were produced according to standard mixing and direct compression methods for tablets coated with pharmaceutically acceptable excipients commonly used in the industry.

In that sense, by way of example, the different components of the formulation of a tablet are mixed in a suitable mixer, for example, a double cone mixing drum, which is rotated at a certain speed, for example 12 rpm, for the time necessary to mix the different components found therein, for example 30 minutes; once it is homogeneous, the mixture is brought to suitable equipment to be transformed into tablets using tools suited to that end, for example, round punches 6 mm in diameter, with the weight indicated in the composition of each tablet, applying a sufficient force to achieve particle cohesion at a suitable thickness, for example 3 - 5 mm

Once obtained, these tablets are coated by applying in suitable equipment, for example a perforated coating drum, a coating agent that has previously been dissolved or dispersed in a suitable liquid vehicle, for example water or purified water; during the coating process, the cores are kept in continuous rotation inside the coating equipment while at the same time the coating agent is sprayed thereon by means of spray guns which atomize the coating suspension, with the coating agent (solid part) being deposited homogeneously on the surface of the tablet forming a continuous layer, while at the same time the liquid vehicle is evaporated by means of the action of a hot air stream.

At the end of the process, the tablets are wrapped in a thin film of coating material, generally 3 - 15% by weight with respect to the total weight of the tablet. Depending on the chemical nature of the solid part of the coating agent, the coating film may impart different properties to the tablets, for example: tablet dissolution at a specific pH such as resistance to an acidic medium (gastro-resistant coating), protection against the oxidation of the components of the tablet as a result of being impermeable to gases such as oxygen, or protection against moisture, and/or an increased resistance to erosion, or improved smell and taste organoleptic characteristics.

The coated tablets were then encapsulated using a specific capsule filling machine, such as for example, a capsule containing a type A tablet, a type B tablet, and two type C tablets.

### Micro granules

Microgranules are multiparticulate systems which are characterized by their shape and size. Microgranules are free-flowing spherical or semispherical agglomerates with a very small particle size distribution generally between 0.5 and 1.5 mm, and even smaller.

The way to obtain these microgranules or pellets can vary from compression (microtablets), dry processes such as hot-melt, to spray drying techniques; however, the most widely used technique is extrusion spheronization.

The extrusion spheronization technique consists of the agglomeration of the sprayed components by means of adding a vehicle (microcrystalline cellulose, starch, lactose, mannitol, hydroxypropyl methylcellulose, or the like) and a binding agent and a humectant (such as for example, HPMC, povidone, and/or polysorbate 80) previously dispersed or dissolved in a liquid vehicle and kneading same in suitable equipment (high shear mixers or sigma or planetary kneading machines). Then, by means of an endless extrusion system, this mass is passed through a multiperforated plate having a specific diameter, for example 1 mm, cutting the obtained cylinders to a specific size, for example 1 - 1.5 mm in length.

The cylinders thus obtained are given a round shape in suitable equipment, for example a spheronizer, with the suitable implement, for example conical plates with a pyramidal relieve of 1 mm, rotating at a high speed, for example 900 - 1500 rpm, for the required time, for example 1-2 minutes.

These wet spheres are dried in a conventional dryer, for example an oven, a drying tunnel, or a fluidized bed dryer until eliminating the residual vehicle of the wet mixes, for example water, and obtaining the suitable consistency.

The spheres thus obtained are sieved to eliminate fines and agglomerates until obtaining the required particle size, for example 0.6 to 1.2 mm.

These spheres can then be coated in suitable equipment, for example smooth or perforated coating equipment, Wurster coating, or the like, in order to wrap the spheres individually in a thin film of coating material generally between 2 and 15% by weight with respect to the total weight of the microgranule.

Depending on the chemical nature of the solid part of the coating agent, the coating film may impart different properties to the microgranules for example: microgranule dissolution at a specific pH such as resistance to an acidic medium (gastro-resistant coating), protection against the oxidation of the components of the microgranule as a result of being impermeable to gases such as oxygen, or protection against moisture, and/or an increased resistance to erosion, or improved smell and taste organoleptic characteristics, or adding other active ingredients on the surface of the microgranule.

The microgranules thus obtained are then metered at a specific weight assuring the dose of the active ingredients to be administered in the capsules for subsequent oral administration.

### Powder

The different components of the formulation are sieved, for example through a mesh with a mesh size of 1 mm, for example, and mixed in a suitable mixer, for example a double cone mixing drum, which is rotated at a certain speed, for example 12 rpm, for the time necessary to mix the different components found therein, such as 30 minutes for example. Once it is homogeneous, the mixture is brought to suitable equipment to be filled into the capsules at the weight defined in the formulation.

### Granulated material

The granulation process consists of the agglomeration of the sprayed components by means of adding a vehicle (microcrystalline cellulose, starch, lactose, mannitol, hydroxypropyl methylcellulose, or the like). It is possible to include an intragranular disintegrating agent (such as for example sodium croscarmellose, modified starch, or the like) to improve the release of the components and a binding agent (such as for example povidone, HPMC, sugar, or the like) previously dispersed or dissolved in a liquid vehicle and the kneading thereof is performed in suitable equipment (high shear rate kneading machines or horizontal or vertical sigma or planetary kneading machines). This mass is wet granulated, forcefully passing it through a mesh with a suitable mesh size, for example 3 - 4 mm.

The wet granulated material thus obtained is dried in a conventional dryer, for example an oven, a drying tunnel, or a fluidized bed dryer until eliminating the residual vehicle of the wet mixes, for example water, and obtaining the suitable consistency.

Once dried, this granulated material is calibrated by passing it through a mesh with a suitable mesh size, for example 1 - 1.2 mm. This obtained granulated material is mixed in a suitable mixer, such as a double cone mixer, for example, rotating at a suitable speed, for example 12 rpm, for the time necessary to homogenize the components of the mixture, for example 15 to 30 minutes, with extragranular excipients, for example lubricants (magnesium stearate, stearic acid, talc, or the like) and disintegrants (sodium croscarmellose, modified starch, or the like).

The granulated material thus obtained is filled into capsules by means of suitable equipment and implement with the weight required for assuring the amount of nutrients indicated in the formulation.

The capsules of the present invention are useful for the treatment of different disorders, such as sleep disorders, anxiety, joint pain, migraines, etc.

### Examples of Formulations

The capsules of the present invention are filled with tablets, microgranules, powder, and/or granulated material of the different food supplements, using a standard capsule filling machine on the market.

The encapsulating system allows adding in one and the same capsule combinations of tablets as well as tablets with another type of product such as microgranules, or powder, and/or granulated material.

Types of tablets to be metered into capsules number 0 with a standard capsule filling machine:

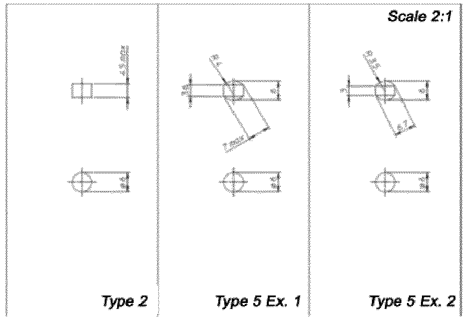

Combinations of tablets in the capsule (type A, B, C, D, E, and F tablets of identical dimensions but different composition)

| No. of tablets | Combinations of tablets in the capsule |
|---|---|
| 3 tablets | Option 1 (2 type A + 1 type B) |
| | Option 2 (1 type A + 1 type B + 1 type C) |
| 4 tablets | Option 1 (2 type A + 1 type B + 1 type C) |
| | Option 2 (3 type A + 1 type B) |
| | Option 3 (2 type A + 2 type B) |
| 5 tablets | Option 1 (2 type A + 2 type B + 1 type C) |
| | Option 2 (3 type A + 1 type B + 1 type C) |
| | Option 3 (1 type A + 1 type B + 1 type C + 1 type D +1 type E) |
| 6 tablets | Option 1 (2 type A + 2 type B + 2 type C) |
| | Option 2 (2 type A + 2 type B + 1 type C + 1 type D) |
| | Option 3 (3 type A + 1 type B + 1 type C + 1 type D) |
| | Option 4 (3 type A + 3 type B) |
| | Option 5 (1 type A + 1 type B + 1 type C + 1 type D +1 type E + 1 type F) |

Preferably, 4 round, type-2, coated tablets with a diameter of 6 mm and a height comprised between 4.1 and 4.4 mm.

### Examples of capsules containing modified-release tablets

### For the treatment of sleep disorder:

Random, double-blind, controlled clinical trials were carried out in parallel groups in patients diagnosed with primary insomnia with a total sleeping time of <6 hours treated with the formulation for insomnia for 2 weeks. During this period, the total sleeping time, sleep latency, and the number of awakenings per night were evaluated. An improvement in the total sleeping time, sleep latency, the number of awakenings per night was observed in patients treated with the supplement.

### Example 1:

The capsule contains four tablets:
i) a coated tablet A containing
   - 0.30 mg melatonin
   - 50 mg GABA
ii) a coated modified-release tablet B containing:
   - 1.65 mg melatonin
   - 50 mg GABA
iii) two coated tablets C containing:
   - 60 mg *Valeriana* extract
   - 60 mg *Passiflora* extract

**Tablet A:**

| | | | |
|---|---|---|---|
| Coated tablet composition containing 0.25 mg melatonin and 50 mg GABA | | | |

| No. | Material | Unit (g) | % |
|---|---|---|---|
| Active ingredients | | | |
| 1 | Melatonin | 0.00030 | 0.194 |
| 2 | GABA | 0.05000 | 32.258 |

| Excipients | | | |
|---|---|---|---|
| 4 | Microcrystalline cellulose | 0.03938 | 25.406 |
| 5 | Calcium hyd. phosphate dihydrate | 0.05369 | 34.639 |
| 6 | Colloidal silica | 0.00078 | 0.503 |
| 7 | Vegetable magnesium stearate | 0.00155 | 1.000 |
| 8 | Sodium starch glycolate | 0.00465 | 3.000 |

| Coating | | | |
|---|---|---|---|
| 9 | PVA, polyethylene glycol, talc, dyes | 0.00465 | 3.000 |
| | Total | 0.155 | 100 |

**Tablet B:**

| Coated modified-release tablet composition containing 1.65 mg melatonin and 50 mg GABA | | | |
|---|---|---|---|
| No. | Material | Unit (g) | % |
| Active ingredients | | | |
| 1 | Melatonin | 0.00165 | 1.065 |
| 2 | GABA | 0.05 | 32.258 |

| Excipients | | | |
|---|---|---|---|
| 3 | Sodium hydroxypropyl methylcellulose | 0.04102 | 26.465 |
| 4 | Calcium hyd. phosphate dihydrate | 0.05613 | 36.213 |
| 5 | Vegetable magnesium stearate | 0.00155 | 1.000 |

| Coating | | | |
|---|---|---|---|
| 6 | PVA, polyethylene glycol, talc, dyes | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |
| Excipients | | | |
| 3 | Microcrystalline cellulose | 0.00773 | 4.833 |
| 4 | Calcium hyd. phosphate dihydrate | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |
| Coating | | | |
| 8 | Eudraguard® control is a 30% aqueous dispersion of neutral methacrylic co-polymer (E 1206, GRAS-status) and calcium alginate | 0.00960 | 6.000 |
| | Total | 0.160 | 100 |

### Example 2:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet com position containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 4 | Microcrystalline cellulose | 0.03938 | 25.403 |
| 5 | Sorbitol | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |

| Coating | | | |
|---|---|---|---|
| 9 | PVA, polyethylene glycol, talc, dyes | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Hydroxypropyl methylcellulose | 0.04102 | 26.462 |
| 4 | Sorbitol | 0.08203 | 52.925 |
| 5 | Vegetable magnesium stearate | 0.00155 | 1.000 |

| Coating | | | |
|---|---|---|---|
| 6 | PVA, polyethylene glycol, talc, dyes | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |

| Excipients | | | |
|---|---|---|---|
| 3 | Microcrystalline cellulose | 0.00773 | 4.833 |
| 4 | Sorbitol | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |

| Coating | | | |
|---|---|---|---|
| 8 | Eudraguard® control is a 30% aqueous dispersion of neutral methacrylic co-polymer (E 1206, GRAS-status) and calcium alginate | 0.00960 | 6.000 |
| | Total | 0.160 | 100 |

### Example 3:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet com position containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 4 | Microcrystalline cellulose | 0.03938 | 25.403 |
| 5 | Mannitol | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |

| Coating | | | |
|---|---|---|---|
| 9 | PVA, polyethylene glycol, talc, dyes | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Hydroxypropyl methylcellulose | 0.04102 | 26.462 |
| 4 | Mannitol | 0.08203 | 52.925 |
| 5 | Vegetable magnesium stearate | 0.00155 | 1.000 |

| Coating | | | |
|---|---|---|---|
| 6 | PVA, polyethylene glycol, talc, dyes | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |

| Excipients | | | |
|---|---|---|---|
| 3 | Microcrystalline cellulose | 0.00773 | 4.833 |
| 4 | Mannitol | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |

| Coating | | | |
|---|---|---|---|
| 8 | Eudraguard® control is a 30% aqueous dispersion of neutral methacrylic co-polymer (E 1206, GRAS-status) | 0.00960 | 6.000 |
| | Total | 0.160 | 100 |

### Example 4:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet com position containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |
| Excipients | | | |
| 4 | Microcrystalline cellulose | 0.03938 | 25.403 |
| 5 | Xylitol | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |
| Coating | | | |
| 9 | PVA, polyethylene glycol, talc, dyes | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |
| Excipients | | | |
| 3 | Hydroxypropyl methylcellulose | 0.04102 | 26.462 |
| 4 | Xylitol | 0.08203 | 52.925 |
| 5 | Vegetable magnesium stearate | 0.00155 | 1.000 |
| Coating | | | |
| 6 | PVA, polyethylene glycol, talc, dyes | 0.00465 | 3.000 |
| | Total | 0.155 | 100.000 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |
| Excipients | | | |
| 3 | Microcrystalline cellulose | 0.00773 | 4.833 |
| 4 | Xylitol | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |
| Coating | | | |
| 8 | Eudraguard® control is a 30% aqueous dispersion of neutral methacrylic co-polymer (E 1206, GRAS-status) | 0.00960 | 6.000 |
| | Total | 0.160 | 100 |

### Example 5:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet composition containing 0.25 mg melatonin and 25 mg GABA | | | |
|---|---|---|---|
| No. | Material | Unit (g) | % |
| Active | | | |
| ingredients | | | |
| 1 | Melatonin | 0.00025 | 0.161 |
| 2 | GABA | 0.02500 | 16.129 |
| Excipients | | | |
| 4 | Microcrystalline cellulose | 0.03938 | 25.403 |
| 5 | Dextrate hydrates | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |
| Coating | | | |
| 9 | PVA, polyethylene glycol, talc, dyes | 0.00465 | 3.000 |
| | Total | 0,155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |
| Excipients | | | |
| 3 | Hydroxypropyl methylcellulose | 0.04102 | 26.462 |
| 4 | Dextrate hydrates | 0.08203 | 52.925 |
| 5 | Vegetable magnesium stearate | 0.00155 | 1.000 |
| Coating | | | |
| 6 | PVA, polyethylene glycol, talc, dyes | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |
| Excipients | | | |
| 3 | Microcrystalline cellulose | 0.00773 | 4.833 |
| 4 | Dextrate hydrates | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |
| Coating | | | |
| 8 | Eudraguard® control is a 30% aqueous dispersion of neutral methacrylic co-polymer (E 1206, GRAS-status) and calcium alginate | 0.00960 | 6.000 |
| | Total | 0.160 | 100 |

### Example 6:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet com position containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |
| Excipients | | | |
| 4 | Microcrystalline cellulose | 0.03938 | 25.403 |
| 5 | Lactose monohydrate | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |
| Coating | | | |
| 9 | PVA, polyethylene glycol, talc, dyes | 0.00465 | 3.000 |
| Total | | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |
| Excipients | | | |
| 3 | Sodium hydroxypropyl methylcellulose | 0.04102 | 26.462 |
| 4 | Lactose monohydrate | 0.08203 | 52.925 |
| 5 | Vegetable magnesium stearate | 0.00155 | 1.000 |
| Coating | | | |
| 6 | PVA, polyethylene glycol, talc, dyes | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | | |
|---|---|---|---|---|
| No. | Material | Unit (g) | % | |
| Active ingredients | | | | |
| 1 | *Valeriana* extract | 0.06000 | 37.500 | |
| 2 | *Passiflora* extract | 0.06000 | 37.500 | |
| Excipients | | | | |
| 3 | Microcrystalline cellulose | 0.00773 | 4.833 | |
| 4 | Lactose Monohydrate | 0.01547 | 9.667 | |
| 5 | Colloidal silica | 0.00080 | 0.500 | |
| 6 | Vegetable magnesium stearate | 0.00160 | 1.000 | |
| 7 | Sodium starch glycolate | 0.00480 | 3.000 | |
| Coating | | | | |
| 8 | Eudraguard® control is a 30% aqueous dispersion of neutral methacrylic co-polymer (E 1206, GRAS-status) and calcium alginate | 0.00960 | 6.000 | |
| | Total | 0.160 | 100 | |

### Example 7:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet composition containing 0.25 mg melatonin and 25 mg GABA | | | |
|---|---|---|---|
| No. | Material | Unit (g) | % |
| Active | | | |
| ingredients | | | |
| 1 | Melatonin | 0.00025 | 0.161 |
| 2 | GABA | 0.02500 | 16.129 |
| Excipients | | | |
| 4 | Microcrystalline cellulose | 0.03938 | 25.403 |
| 5 | Lactose monohydrate | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |
| Coating | | | |
| 9 | HPMC 2910/Hypromellose, Titanium Dioxide, Macrogol/PEG | 0.00465 | 3.000 |
| | Total | 0.155 | 100 |

**Tablet B:**

| | | | |
|---|---|---|---|
| Coated modified-re release tablet composition containing 0.75 mg melatonin and 25 mg GABA | | | |
| No. | Material | Unit (g) | % |
| Active | | | |
| ingredients | | | |
| 1 | Melatonin | 0.00075 | 0.484 |
| 2 | GABA | 0.02500 | 16.129 |
| Excipients | | | |
| 3 | Sodium hydroxypropyl | 0.04102 | 26.462 |
| | methylcellulose | | |
| 4 | Lactose monohydrate | 0.08203 | 52.925 |
| 5 | Vegetable magnesium stearate | 0.00155 | 1.000 |
| Coating | | | |
| | HPMC 2910/Hypromellose, | | |
| 6 | Titanium Dioxide, Macrogol/PEG | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |
| Excipients | | | |
| 3 | Microcrystalline cellulose | 0.00773 | 4.833 |
| 4 | Lactose monohydrate | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |
| Coating | | | |
| 8 | Triethyl citrate (E-1505), glycerol monostearate, polysorbate 80 (E-433), simethicone, 30% dispersion of methacrylic acid and ethyl acrylate co-polymer (1:1) and calcium alginate | 0.00960 | 6.000 |
| | Total | 0.160 | 100 |

### Example 8:

The capsule contains four tablets:
i) a coated tablet A containing
   - 0.25 mg melatonin
   - 25 mg GABA
ii) a coated modified-release tablet B containing:
   - 0.75 mg melatonin
   - 25 mg GABA
iii) two coated tablets C containing:
   - 60 mg of *Valeriana* extract
   - 60 mg of *Passiflora* extract

**Tablet A:**

| Coated tablet composition containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 4 | Microcrystalline cellulose | 0.03938 | 25.403 |
| 5 | Hydroxypropyl cellulose (E-463) | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |

| Coating | | | |
|---|---|---|---|
| 9 | HPMC 2910/Hypromellose, Titanium Dioxide, Macrogol/PEG | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Sodium hydroxypropyl methylcellulose | 0.04102 | 26.462 |
| 4 | Hydroxypropyl cellulose (E-463) | 0.08203 | 52.925 |
| 5 | Vegetable magnesium stearate | 0.00155 | 1.000 |

| Coating | | | |
|---|---|---|---|
| 6 | HPMC 2910/Hypromellose, Titanium Dioxide, Macrogol/PEG | 0.00465 | 3.000 |
| | Total | 0.155 | 100 |

**Tablet C:**

| Coated tablet composition containing 60 mg *Valeriana* extract and 60 mg *Passiflora* extract with gastro-resistant coating: | | | |
|---|---|---|---|
| No. | Material | Unit (g) | % |
| Active ingredients | | | |
| 1 | *Valeriana* extract | 0.06000 | 37.500 |
| 2 | *Passiflora* extract | 0.06000 | 37.500 |

| Excipients | | | |
|---|---|---|---|
| 3 | Microcrystalline cellulose | 0.00773 | 4.833 |
| 4 | Hydroxypropyl cellulose (E-463) | 0.01547 | 9.667 |
| 5 | Colloidal silica | 0.00080 | 0.500 |
| 6 | Vegetable magnesium stearate | 0.00160 | 1.000 |
| 7 | Sodium starch glycolate | 0.00480 | 3.000 |

| Coating | | | |
|---|---|---|---|
| 8 | Triethyl citrate (E-1505), glycerol monostearate, polysorbate 80 (E-433), simethicone, 30% dispersion of methacrylic acid and ethyl acrylate co-polymer (1:1) | 0.00960 | 6.000 |
| | Total | 0.160 | 100 |

### Example 9:

The gelatin or HPMC capsule contains:
- 160 mg of sustained-release microgranules with gastro-resistant coating
- 1 Type A tablet
- 1 Type B tablet

### Microgranules

Microgranule composition per capsule containing 60 mg *Valeriana* extract and 60 mg *Passiflora* extract with gastro-resistant coating:

| No. | Material | Unit (g) | % |
|---|---|---|---|
| Active ingredients | | | |
| 1 | *Valeriana* extract | 0.0600 | 37.5 |
| 2 | *Passiflora* extract | 0.0600 | 37.5 |

| Excipients | | | |
|---|---|---|---|
| 3 | Hydroxypropyl cellulose (E-463) | 0.0032 | 2 |
| 4 | Mannitol | 0.0192 | 12 |
| 5 | Lactose Monohydrate | 0.0032 | 2 |
| 6 | Sodium starch glycolate | 0.0016 | 1 |
| 7 | Polysorbate 80 | 0.0016 | 1 |
| 8 | Povidone K 29-32 | 0.0016 | 1 |

| Coating | | | |
|---|---|---|---|
| 9 | Triethyl citrate (E-1505), glycerol monostearate, polysorbate 80 (E-433), simethicone, 30% dispersion of methacrylic acid and ethyl acrylate co-polymer (1:1) | 0.0096 | 6 |
| | | 0.160 | 100.000 |

**Tablet A:**

| Coated tablet com | position containing 0.25 mg melatonin and 25 mg GABA | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 4 | Microcrystalline cellulose | 0.03938 | 25.403 |
| 5 | Hydroxypropyl cellulose (E-463) | 0.07875 | 50.806 |
| 6 | Colloidal silica | 0.00078 | 0.500 |
| 7 | Vegetable magnesium stearate | 0.00155 | 1.000 |
| 8 | Sodium starch glycolate | 0.00465 | 3.000 |

| Coating | | | |
|---|---|---|---|
| 9 | HPMC 2910/Hypromellose, Titanium Dioxide, Macrogol/PEG | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Sodium hydroxypropyl methylcellulose | 0.04102 | 26.462 |
| 4 | Hydroxypropyl cellulose (E-463) | 0.08203 | 52.925 |
| 5 | Vegetable magnesium stearate | 0.00155 | 1.000 |

| Coating | | | |
|---|---|---|---|
| 6 | HPMC 2910/Hypromellose, Titanium Dioxide, Macrogol/PEG | 0.00465 | 3.000 |
| | Total | 0.155 | 100 |

### Example 10:

The gelatin or HPMC capsule contains:
- 160 mg of powder
- 1 Type A tablet
- 1 Type B tablet

### Powder:

Powder composition per capsule containing 60 mg *Valeriana* extract and 60 mg *Passiflora* extract:

| No. | Material | Unit (g) | % |
|---|---|---|---|
| Active ingredients | | | |
| 1 | *Valeriana* extract | 0.06 | 37.5 |
| 2 | *Passiflora* extract | 0.06 | 37.5 |

| Excipients | | | |
|---|---|---|---|
| 3 | Lactose monohydrate | 0.0392 | 24.5 |
| 4 | Magnesium stearate | 0.0008 | 0.5 |
| | Total | 0.160 | 100 |

**Tablet A:**

| Coated tablet composition | containing 0.25 mg melatonin and 25 mg GABA | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 3 | Microcrystalline cellulose | 0.03938 | 25.403 |
| 4 | Hydroxypropyl cellulose (E-463) | 0.07875 | 50.806 |
| 5 | Colloidal silica | 0.00078 | 0.500 |
| 6 | Vegetable magnesium stearate | 0.00155 | 1.000 |
| 7 | Sodium starch glycolate | 0.00465 | 3.000 |

| Coating | | | |
|---|---|---|---|
| 8 | HPMC 2910/Hypromellose, | 0.00465 | 3.000 |
| | Titanium Dioxide, Macrogol/PEG | | |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Sodium hydroxypropyl methylcellulose | 0.04102 | 26.462 |
| 4 | Hydroxypropyl cellulose (E-463) | 0.08203 | 52.925 |
| 5 | Vegetable magnesium stearate | 0.00155 | 1.000 |

| Coating | | | |
|---|---|---|---|
| 6 | HPMC 2910/Hypromellose, Titanium Dioxide, Macrogol/PEG | 0.00465 | 3.000 |
| | Total | 0.155 | 100 |

### Example 11:

The gelatin or HPMC capsule contains:
- 160 mg of granulated material
- 1 Type A tablet
- 1 Type B tablet

### Granulated material:

Granulated material composition per capsule containing 60 mg *Valeriana* extract and 60 mg *Passiflora* extract:

| No. | Material | Unit (g) | % |
|---|---|---|---|
| Active ingredients | | | |
| 1 | *Valeriana* extract | 0.06000 | 38.710 |
| 2 | *Passiflora* extract | 0.06000 | 38.710 |

| Excipients | | | |
|---|---|---|---|
| 3 | Microcrystalline cellulose | 0.00573 | 3.694 |
| 4 | Mannitol | 0.01145 | 7.387 |
| 5 | PVP | 0.00775 | 5.000 |
| 6 | Vegetable magnesium stearate | 0.00078 | 0.500 |
| 7 | Sodium starch glycolate | 0.00465 | 3.000 |

| Coating | | | |
|---|---|---|---|
| 8 | PVA, polyethylene glycol, talc, dyes | 0.00465 | 3.000 |
| | Total | 0.155 | 100 |

**Tablet A:**

| Coated tablet composition containing 0.25 mg melatonin and 25 mg GABA | | | |
|---|---|---|---|
| No. | Material | Unit (g) | % |
| Active ingredients | | | |
| 1 | Melatonin | 0.00025 | 0.161 |
| 2 | GABA | 0.02500 | 16.129 |

| Excipients | | | |
|---|---|---|---|
| 3 | Microcrystalline cellulose | 0.03938 | 25.403 |
| 4 | Hydroxypropyl cellulose (E-463) | 0.07875 | 50.806 |
| 5 | Colloidal silica | 0.00078 | 0.500 |
| 6 | Vegetable magnesium stearate | 0.00155 | 1.000 |
| 7 | Sodium starch glycolate | 0.00465 | 3.000 |

| Coating | | | |
|---|---|---|---|
| 8 | HPMC 2910/Hypromellose, Titanium Dioxide, Macrogol/PEG | 0.00465 | 3.000 |
| | Total | 0.155 | 100 |

**Tablet B:**

| Coated modified-release tablet composition containing 0.75 mg melatonin and 25 mg GABA | | | |
|---|---|---|---|
| No. | Material | Unit (g) | % |
| Active ingredients | | | |
| 1 | Melatonin | 0.00075 | 0.484 |
| 2 | GABA | 0.02500 | 16.129 |

| Excipients | | | |
|---|---|---|---|
| 3 | Sodium hydroxypropyl methylcellulose | 0.04102 | 26.462 |
| 4 | Hydroxypropyl cellulose (E-463) | 0.08203 | 52.925 |
| 5 | Vegetable magnesium stearate | 0.00155 | 1.000 |

| Coating | | | |
|---|---|---|---|
| 6 | HPMC 2910/Hypromellose, Titanium Dioxide, Macrogol/PEG | 0.00465 | 3.000 |
| Total | | 0.155 | 100 |

### For the treatment of anxiety

The formulation for anxiety object of the patent containing 5-HTP, vitamin B6, and magnesium was evaluated in comparison with a selective serotonin reuptake inhibitor (SSRI) drug in a multicenter, double-blind study design. A total of 36 subjects, all of whom were diagnosed with some form of depression, received the formulation for anxiety object of the patent or an antidepressant three times a day. The subjects were evaluated at 0, 2, 4, and 6 weeks using four evaluation tools: the Hamilton Rating Scale for Depression (HRSD), a standard scale for depression rating; a self-evaluation performed by the patient; a severity evaluation performed by the investigator, and a clinical global impression. Both treatment groups showed significant, almost identical reductions in depression after the second week and these reductions continued up to the sixth week. After four weeks, 15 of the 36 patients treated with 5-HTP and 18 of the 33 patients treated with the antidepressant had improved by at least 50%, according to HRSD scores. In week six, the two groups had about the same number showing a 50% improvement. When the numbers were added up at the end of the study, the investigators found that the average percentage improvement from the baseline to the final evaluation was slightly higher (at 5%) for patients treated for anxiety with the formulation object of the patent.

### Example 12:

The gelatin or HPMC capsule contains four tablets in a capsule:
- 1 coated modified-release tablet containing kava-kava (amount of extract equivalent to 100 mg of kavalactones)
- 2 coated tablets containing: 100 mg magnesium
- 1 coated modified-release tablet containing: 26 mg vitamin B6

**Tablet A:**

| Coated tablet | composition containing 100 mg kava-kava: | | |
|---|---|---|---|
| No. | Material | Unit (g) | % |
| Active ingredients | | | |
| 1 | Kava-Kava | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, | 0.0063 | 3.000 |
| | dyes | | |
| | Total | 0.209 | 100 |

**Tablet B:**

| Coated tablet composition containing 100 mg magnesium: | | | |
|---|---|---|---|
| No. | Material | Unit (g) | % |
| Active ingredients | | | |
| 1 | Magnesium oxide | 0.1660 | 79.426 |

| Excipients | | | |
|---|---|---|---|
| 2 | Microcrystalline cellulose 102 | 0.0063 | 3.025 |
| 3 | Calcium hyd. phosphate anhyd. | 0.0126 | 6.049 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

**Tablet C:**

| Coated modified-release tablet composition containing 26 mg vitamin B6: | | | |
|---|---|---|---|
| No. | Material | Unit (g) | % |
| Active ingredients | | | |
| 1 | Pyridoxine hydrochloride | 0.0312 | 14.928 |

| Excipients | | | |
|---|---|---|---|
| 2 | HPMC | 0.0513 | 24.524 |
| 3 | Calcium hyd. phosphate anhyd. | 0.1025 | 49.048 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, | 0.0063 | 3.000 |
| | talc, dyes | | |
| | Total | 0.209 | 100 |

### Example 13:

The gelatin or HPMC capsule contains four tablets in a capsule:
- 1 coated modified-release tablet containing 100 mg 5-hydroxytryptophan (5-HTP)
- 1 coated modified-release tablet containing 10 mg vitamin B6
- 2 coated tablets containing 100 mg magnesium

**Tablet A:**

| Coated modified-release tablet composition containing 100 mg 5-HTP: | | | |
|---|---|---|---|
| No. | Material | Unit (g) | % |
| Active ingredients | | | |
| 1 | 5-HTP | 0.1000 | 47.847 |

| Excipients | | | |
|---|---|---|---|
| 2 | Calcium hyd. phosphate dihydrate | 0.0262 | 12.551 |
| 3 | Microcrystalline cellulose 102 | 0.0525 | 25.102 |
| 4 | HPMC | 0.0209 | 10.000 |
| 5 | Colloidal silica | 0.0010 | 0.500 |
| 6 | Vegetable magnesium stearate | 0.0021 | 1.000 |

| Coating | | | |
|---|---|---|---|
| 7 | PVA, polyethylene glycol, talc, dyes | 0.0063 | 3.000 |
| | Total | 0.209 | 100 |

**Tablet B:**

| Coated modified-release tablet composition containing 10 mg vitamin B6: | | | |
|---|---|---|---|
| No. | Material | Unit (g) | % |
| Active ingredients | | | |
| 1 | Pyridoxine hydrochloride | 0.0120 | 5.742 |

| Excipients | | | |
|---|---|---|---|
| 2 | Calcium hyd. phosphate dihydrate | 0.0556 | 26.586 |
| 3 | Microcrystalline cellulose PH102 | 0.1111 | 53.172 |
| 4 | HPMC | 0.0209 | 10.000 |
| 5 | Colloidal silica | 0.0010 | 0.500 |
| 6 | Vegetable magnesium stearate | 0.0021 | 1.000 |

| Coating | | | |
|---|---|---|---|
| 7 | PVA, polyethylene glycol, talc, | 0.0063 | 3.000 |
| | dyes | | |
| | Total | 0.209 | 100 |

**Tablet C:**

| Coated tablet composition containing 100 mg magnesium: | | | |
|---|---|---|---|
| No. | Material | Unit (g) | % |
| Active ingredients | | | |
| 1 | Magnesium oxide | 0.1660 | 79.426 |

| Excipients | | | |
|---|---|---|---|
| 2 | Microcrystalline cellulose 102 | 0.0063 | 3.025 |
| 3 | Calcium hyd. phosphate anhyd. | 0.0126 | 6.049 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

### Example 14:

The gelatin or HPMC capsule contains four tablets in a capsule:
- 1 coated modified-release tablet containing 28 mg dry saffron extract, 10 mg 5-hydroxytryptophan (5-HTP)
- 1 coated modified-release tablet containing 10 mg vitamin B6
- 2 coated tablets containing 60 mg magnesium

**Tablet A:**

| Coated modified-release tablet composition containing 28 mg saffron and 10 mg 5-HTP | | | |
|---|---|---|---|
| No. | Material | Unit (g) | % |
| Active ingredients | | | |
| 1 | 5-HTP | 0.0100 | 7.348 |
| 2 | Saffron (*Crocus sativus L*.) extract | 0.0280 | 20.573 |

| Excipients | | | |
|---|---|---|---|
| 2 | Calcium hyd. phosphate dihydrate | 0.0302 | 22.190 |
| 3 | Microcrystalline cellulose 102 | 0.0605 | 44.453 |
| 4 | Colloidal silica | 0.0007 | 0.514 |
| 5 | Vegetable magnesium stearate | 0.0007 | 0.514 |

| Modified -release sealing coating | | | |
|---|---|---|---|
| 6 | Shellac | 0.0020 | 1.470 |

| Color coating | | | |
|---|---|---|---|
| 7 | Hydroxypropyl methylcellulose, Microcrystalline cellulose, stearic acid, Dye | 0.0040 | 2.939 |
| | Total | 0.136 | 100 |

**Tablet B:**

| Coated modified-release tablet composition containing 10 mg vitamin B6: | | | |
|---|---|---|---|
| No. | Material | Unit (g) | % |
| Active ingredients | | | |
| 1 | Pyridoxine hydrochloride | 0.0120 | 9.639 |

| Excipients | | | |
|---|---|---|---|
| 2 | Microcrystalline cellulose PH102 | 0.0922 | 74.056 |
| 3 | HPMC | 0.0152 | 12.209 |
| 4 | Vegetable magnesium stearate | 0.0006 | 0.482 |

| Modified -release sealing coating | | | |
|---|---|---|---|
| 5 | Shellac | 0.0020 | 1.606 |

| Color coating | | | |
|---|---|---|---|
| 6 | Hydroxypropyl methylcellulose, TiO₂, hypromellose, glycerin, dye | 0.0025 | 2.008 |
| | Total | 0.125 | 100 |

**Tablet C:**

| Coated tablet composition containing 60 mg magnesium: | | | |
|---|---|---|---|
| No. | Material | Unit (g) | % |
| Active ingredients | | | |
| 1 | Magnesium oxide | 0.0996 | 59.286 |

| Excipients | | | |
|---|---|---|---|
| 2 | Microcrystalline cellulose 102 | 0.053 | 31.548 |
| 3 | Co-processed colloidal silica, hydroxypropyl methylcellulose and sodium carboxymethyl cellulose | 0.0116 | 6.905 |
| 4 | Vegetable magnesium stearate | 0.0008 | 0.476 |

| Coating | | | |
|---|---|---|---|
| 5 | Polyvinyl alcohol, TiO₂, Macrogol, talc | 0.003 | 1.786 |
| | Total | 0.120 | 100 |

### For the treatment of migraine (Modified-release):

By means of a single-blind clinical trial conducted in patients with migraines assigned randomly into two parallel groups, the efficacy of a treatment based on the food supplement object of the patent with respect to a placebo was determined. The first group was subjected to treatment with the supplement based on vitamin B2, CoQ10, and magnesium object of the patent, and the second group was treated with placebo. The patients were examined at the beginning of the study and 4, 8, and 12 weeks after. After the administration of the products in both groups, the frequency of migraine episodes and the severity of headache, as well as a self-evaluation of the efficacy performed by the patient, were recorded.

### Result Summary:

The days with migraines per month were reduced from 6.2 days during the initial period to 4.4 days after 12 weeks of treatment with the food supplement and from 6.2 to 5.2 days in the group treated with placebo (p = 0.23).

The intensity of headaches was reduced significantly in the group treated with the food supplement compared with the group treated with placebo (p = 0.03).

Efficacy evaluation by the patient was better in the group treated with the supplement with respect to placebo (p = 0.01).

### Example 15:

The hypromellose capsule no. 0 contains four tablets:
- 1 coated tablet containing 100 mg coenzyme Q-10
- 1 coated modified-release tablet with enteric coating containing:
   - 100 mg vitamin B2
   - 25 mg citric acid
- 2 coated tablets containing 100 mg magnesium

**Tablet A:**

| Coated tablet composition containing 100 mg coenzyme Q10: | | | |
|---|---|---|---|
| No. | Material | Unit (g) | % |
| Active ingredients | | | |
| 1 | Coenzyme Q10 | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

**Tablet B:**

| Coated modified-release tablet composition with gastro-resistant coating containing 100 mg vitamin B2 and 25 mg citric acid: | | | |
|---|---|---|---|
| No. | Material | Unit (g) | % |
| Active ingredients | | | |
| 1 | Riboflavin | 0.1000 | 47.170 |
| 2 | Citric acid | 0.0250 | 11.792 |

| Excipients | | | |
|---|---|---|---|
| 3 | Calcium hyd. phosphate dihydrate | 0.0166 | 7.846 |
| 4 | Microcrystalline cellulose 102 | 0.0333 | 15.692 |
| 5 | HPMC | 0.0212 | 10.000 |
| 6 | Colloidal silica | 0.0011 | 0.500 |
| 7 | Vegetable magnesium stearate | 0.0021 | 1.000 |

| Coating | | | |
|---|---|---|---|
| 8 | Triethyl citrate (E-1505), glycerol monostearate, polysorbate 80 (E-433), simethicone, 30% dispersion of methacrylic acid and ethyl acrylate co-polymer (1:1) and calcium alginate | 0.0127 | 6.000 |
| | Total | 0.212 | 100 |

**Tablet C:**

| Coated tablet composition containing 100 mg magnesium: | | | |
|---|---|---|---|
| No. | Material | Unit (g) | % |
| Active ingredients | | | |
| 1 | Magnesium oxide | 0.1660 | 79.426 |

| Excipients | | | |
|---|---|---|---|
| 2 | Microcrystalline cellulose 102 | 0.0063 | 3.025 |
| 3 | Calcium hyd. phosphate anhyd. | 0.0126 | 6.049 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

### Antioxidant (Modified-release):

### Example 16:

The hypromellose capsule no. 0 contains four tablets:
- 1 coated tablet containing 100 mg resveratrol
- 1 coated tablet containing 100 mg rosehip extract
- 1 coated tablet containing 100 mg acai
- 1 tablet with enteric coating containing 100 mg quercetin.

This formulation combines natural ingredients with high antioxidant power and a modified release to assure the duration of the effect.

**Tablet A:**

| Coated tablet composition containing 100 mg resveratrol: | | | |
|---|---|---|---|
| No. | Material | Unit (g) | % |
| Active ingredients | | | |
| 1 | Resveratrol | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica Vegetable magnesium | 0.0010 | 0.500 |
| 5 | stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

**Tablet B:**

| Coated tablet composition containing 100 mg rosehip extract: | | | |
|---|---|---|---|
| No. | Material | Unit (g) | % |
| Active ingredients | | | |
| 1 | Rosehip extract | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

**Tablet C:**

| Coated tablet composition containing 100 mg acai: | | | |
|---|---|---|---|
| No. | Material | Unit (g) | % |
| Active ingredients | | | |
| 1 | Acai extract | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica | 0.0010 | 0.500 |
| 5 | Vegetable magnesium stearate | 0.0021 | 1.000 |
| 6 | Sodium starch glycolate | 0.0146 | 7.000 |

| Coating | | | |
|---|---|---|---|
| 7 | PVA, polyethylene glycol, talc, dyes | 0.0063 | 3.000 |
| | Total | 0.209 | 100 |

**Tablet D:**

| Composition tablet coated with gastro-resistant coating containing 100 mg quercetin: | | | |
|---|---|---|---|
| No. | Material | Unit (g) | % |
| Active ingredients | | | |
| 1 | Quercetin | 0.1000 | 47.847 |

| Excipients | | | |
|---|---|---|---|
| 2 | Microcrystalline cellulose 102 | 0.0283 | 13.551 |
| 3 | Calcium hyd. phosphate anhyd. | 0.0566 | 27.102 |
| 4 | Colloidal silica | 0.0010 | 0.500 |
| 5 | Vegetable magnesium stearate | 0.0021 | 1.000 |
| 6 | Sodium starch glycolate | 0.0084 | 4.000 |

| Coating | | | |
|---|---|---|---|
| 7 | Triethyl citrate (E-1505), glycerol monostearate, polysorbate 80 (E-433), simethicone, 30% dispersion of methacrylic acid and ethyl acrylate co-polymer (1:1) and calcium alginate | 0.0126 | 6.000 |
| | Total | 0.209 | 100 |

### For the treatment of joint pain (Modified-release):

The efficacy of the formulation with turmeric, piperine, eggshell membrane, chondroitin sulfate, glucosamine, and vitamin C was evaluated in 50 individuals affected by OA (diagnosis confirmation by means of x-ray). The symptoms were evaluated by means of the WOMAC index; mobility was studied by means of treadmill walking performance; and the overall inflammatory response function was evaluated by means of measuring the plasma concentration of reactive protein C. The trial was carried out for three months, the individuals were randomly divided into two groups receiving, respectively, the supplement object of the patent (in two separate administrations a day) and the "best treatment available", or the "best treatment" alone, as defined by professionals or specialists. The treadmill performance (10% inclination, speed 3 km/h) showed a 201% improvement in the initial walking distance after two months and an improvement (+ 44%) three months after the start of the study. These positive results were complemented by secondary observations, i.e., a decrease in rescue rate with additional medication (63% in the group with supplement with respect to 12% in the group with pharmacological treatment) and a decrease in gastrointestinal complications (38% in the group with supplement vs. 15% in controls (p <0.05).

The following table summarizes the obtained results:

| Parameter | | |
|---|---|---|
| Treadmill walking distance | 201% improvement after 2 months | 44% final improvement |
| Decrease in rescue rate | 63% group with supplement | 12% pharmacologically treated group |
| Decrease in gastrointestinal complications | 38% group with supplement | 15% control group |

This shows a significant improvement in the group treated with the supplement object of the patent with respect to the control group.

### Example 17:

### Night capsule (regenerative effect)

The capsule contains 4 tablets:
- 2 tablets A x 50 mg chondroitin Sulfate
- 1 tablet B x 30.6 mg glucosamine
- 1 sustained-release tablet C x 41 mg vitamin C

### Day capsule (anti-inflammatory effect):

The capsule contains 4 tablets:
- 1 tablet A x 100 mg Ovomet® Eggshell Membrane (pure egg membrane obtained through a production process patented by Eggnovo)
- 2 sustained-release tablets B x 100 mg 12 h Ovomet® Eggshell Membrane
- 1 sustained-release tablet C x 105 mg 95% turmeric + 1 mg 12 h piperine and gastro-resistant coating

### Night capsule (regenerative effect)

**Tablet A:**

| Coated tablet composition | containing 50 mg chondroitin sulfate | | |
|---|---|---|---|
| No. | Material | Unit (g) | % |
| Active ingredients | | | |
| 1 | Chondroitin sulfate | 0.05000 | 32.258 |
| Excipients | | | |
| 2 | Microcrystalline cellulose | 0.03113 | 20.081 |
| 3 | Calcium hyd. phosphate dihydrate | 0.06225 | 40.161 |
| 4 | Colloidal silica | 0.00078 | 0.500 |
| 5 | Vegetable magnesium stearate | 0.00155 | 1.000 |
| 6 | Sodium starch glycolate | 0.00465 | 3.000 |
| Coating | | | |
| 7 | PVA, polyethylene glycol, talc, dyes | 0.00465 | 3.000 |
| | Total | 0.155 | 100 |

**Tablet B:**

| Coated tablet composition | containing 30.6 mg glucosamine: | | |
|---|---|---|---|
| No. | Material | Unit (g) | % |
| Active ingredients | | | |
| 1 | Glucosamine | 0.03060 | 19.742 |
| Excipients | | | |
| 2 | Microcrystalline cellulose | 0.03759 | 24.253 |
| 3 | Calcium hyd. phosphate dihydrate | 0.07518 | 48.505 |
| 4 | Colloidal silica | 0.00078 | 0.500 |
| 5 | Vegetable magnesium stearate | 0.00155 | 1.000 |
| 6 | Sodium starch glycolate | 0.00465 | 3.000 |
| Coating | | | |
| 7 | PVA, polyethylene glycol, talc, dyes | 0.00465 | 3.000 |
| | Total | 0.155 | 100 |

**Tablet C:**

| Coated sustained-release tablet composition containing 41 mg vitamin C: | | | |
|---|---|---|---|
| No. | Material | Unit (g) | % |
| Active ingredients | | | |
| 1 | Vitamin C | 0.04100 | 26.452 |
| Excipients | | | |
| 2 | Hydroxypropyl methylcellulose Calcium hyd. phosphate dihydrate | 0.03593 0.07187 | 23.183 46.366 |
| 3 | Vegetable magnesium stearate | 0.00155 | 1.000 |
| Coating | | | |
| 4 | PVA, polyethylene glycol, talc, dyes | 0.00465 | 3.000 |
| | Total | 0.155 | 100 |

### Day capsule (anti-inflammatory effect):

**Tablet A:**

| Coated tablet composition containing 100 mg egg membrane (OVOMED®) | | | |
|---|---|---|---|
| No. | Material | Unit (g) | % |
| Active ingredients | | | |
| 1 | Egg membrane | 0.10000 | 46.51163 |
| Excipients | | | |
| 2 | Calcium hyd. phosphate dihydrate | 0.03009 | 13.99612 |
| 3 | Microcrystalline cellulose 102 | 0.06018 | 27.99225 |
| 4 | Sodium starch glycolate | 0.01505 | 7.00000 |
| 5 | Colloidal silica | 0.00108 | 0.50000 |
| 6 | Vegetable magnesium stearate | 0.00215 | 1.00000 |
| Coating | | | |
| 7 | PVA, polyethylene glycol, talc, dyes | 0.00645 | 3.00000 |
| | Total | 0.215 | 100.00 |

**Tablet B:**

| Coated sustained-release tablet composition containing 100 mg egg membrane (OVOMET®): | | | |
|---|---|---|---|
| No. | Material | Unit (g) | % |
| Active ingredients | | | |
| 1 | Egg membrane | 0.10000 | 46.51163 |
| Excipients | | | |
| 2 | Calcium hyd. phosphate dihydrate | 0.02794 | 12.99612 |
| 3 | Microcrystalline cellulose 102 | 0.05588 | 25.99225 |
| 4 | HPMC | 0.02150 | 10.00000 |
| 5 | Colloidal silica | 0.00108 | 0.50000 |
| 6 | Vegetable magnesium stearate | 0.00215 | 1.00000 |
| Coating | | | |
| 7 | PVA, polyethylene glycol, talc, dyes | 0.00645 | 3.00000 |
| | Total | 0.215 | 100.00 |

**Tablet C:**

| Coated modified-release tablet composition with gastro-resistant coating containing 105 mg 95% turmeric + 1 mg piperine: | | | |
|---|---|---|---|
| No. | Material | Unit (g) | % |
| Active ingredients | | | |
| 1 | Turmeric | 0.10530 | 48.97674 |
| 2 | Piperine | 0.00105 | 0.48837 |
| Excipients | | | |
| 3 | Calcium hyd. phosphate dihydrate | 0.02583 | 12.01163 |
| 4 | Microcrystalline cellulose PH102 | 0.05165 | 24.02326 |
| 5 | HPMC | 0.02150 | 10.00000 |
| 6 | Colloidal silica | 0.00108 | 0.50000 |
| 7 | Vegetable magnesium stearate | 0.00215 | 1.00000 |
| Coating | | | |
| 8 | Triethyl citrate (E-1505), glycerol monostearate, polysorbate 80 (E-433), simethicone, 30% dispersion of methacrylic acid and ethyl acrylate co-polymer (1:1) and calcium alginate triethyl citrate (E-1505), glycerol monostearate, polysorbate 80 (E-433), simethicone, 30% dispersion of methacrylic acid and ethyl acrylate co-polymer (1:1) and calcium alginate | 0.01332 | 6.00000 |
| | Total | 0.222 | 100.00 |

### Helping the normal functioning of the immune system

### Example 18:

The gelatin or HPMC capsule contains four tablets in a capsule:
- 1 coated modified-release tablet containing 50 meg vitamin D (2000 IU) and 38 mcg vitamin K2
- 1 coated modified-release tablet containing 14 mg iron and 12 mg vitamin C
- 2 coated tablets containing 50 mg dry *Echinacea angustifolia* extract (4% echinosides)

**Tablet A:**

| Coated modified-release tablet composition containing 50 mcg vitamin D (2000 IU) and 38 mcg vitamin K2: | | | | |
|---|---|---|---|---|
| No. | Material | Unit (g) | % | |
| Active ingredients | | | | |
| 1 | Vitamin D3 (0.25%) | 0.0200 | 14.9 | |
| 2 | Vitamin K2 Mk-7 (0.2%) | 0.0190 | 14.2 | |
| Excipients | | | | |
| 3 | Calcium hyd. phosphate dihydrate | 0.0299 | 22.3 | |
| 4 | Microcrystalline cellulose 102 | 0.0598 | 44.6 | |
| 5 | Colloidal silica | 0.0007 | 0.5 | |
| 6 | Magnesium stearate | 0.0007 | 0.5 | |
| Modified-release sealing coating | | | | |
| 7 | Shellac | 0.0013 | 1.0 | |
| Color coating | | | | |
| 8 | Hydroxypropyl methylcellulose, microcrystalline cellulose, stearic acid, dye | 0.0026 | 1.9 | |
| | | Total | 0.134 | 100 |

**Tablet B:**

| • Coated modified-r elease tablet composition containing 14 mg iron and 12 mg vitamin C: | | | |
|---|---|---|---|
| No. | Material | Unit (g) | % |
| Active ingredients | | | |
| 1 | Coated ascorbic acid (97.5%) Ferric saccharate | 0.0123 | 9.1 |
| 2 | microencapsulated in calcium alginate (40%) | 0.0350 | 25.8 |
| Excipients | | | |
| 2 | Microcrystalline cellulose PH102 | 0.0697 | 51.5 |
| 3 | Calcium hyd. phosphate dihydrate | 0.0078 | 5.8 |
| 4 | HPMC | 0.0039 | 2.9 |
| 5 | Colloidal silica | 0.0007 | 0.5 |
| 6 | Magnesium stearate | 0.0007 | 0.5 |
| Modified -release sealing coating | | | |
| 7 | Shellac | 0.0026 | 1.9 |
| Color coating | | | |
| 8 | Hydroxypropyl methylcellulose, TiO₂, hypromellose, glycerin, dye | 0.0027 | 2.0 |
| | Total | 0.135 | 100 |

**Tablet C:**

| Coated tablet composition containing 50 mg dry *Echinacea angustifolia* extract (4% echinosides) : | | | |
|---|---|---|---|
| No. | Material | Unit (g) | % |
| Active ingredients | | | |
| | *Echinacea angustifolia* (4% | | |
| 1 | echinosides) | 0.0500 | 37.5 |
| Excipients | | | |
| 2 | Microcrystalline cellulose 102 | 0.0500 | 37.5 |
| 3 | Calcium hyd. phosphate dihydrate | 0.0250 | 18.7 |
| 4 | Sodium starch glycolate | 0.0039 | 2.9 |
| 5 | Colloidal silica | 0.0007 | 0.5 |
| 6 | Vegetable magnesium stearate | 0.0007 | 0.5 |
| Sealing coating | | | |
| 7 | Shellac | 0.0006 | 0.4 |
| Color coating | | | |
| 8 | Polyvinyl alcohol, TiO₂, Macrogol, talc, dye | 0.0026 | 1.9 |
| | Total | 0.134 | 100 |

### Helping the normal functioning of the eye

### Example 19:

The gelatin or HPMC capsule contains four tablets in a capsule:
- 1 coated modified-release tablet containing 4 mg astaxanthin.
- 1 coated modified-release tablet containing 14 mg zinc and 1 mg copper.
- 2 coated tablets containing 5 mg lutein and 1 mg zeaxanthin

**Tablet A:**

| • Coated modified-release tablet composition containing 4 mg astaxanthin: | | | |
|---|---|---|---|
| No. | Material | Unit (g) | % |
| Active ingredients | | | |
| 1 | Astaxanthin (10%) | 0.0400 | 28.4 |
| Excipients | | | |
| 2 | Microcrystalline cellulose | 0.0283 | 20.1 |
| 3 | Calcium hyd. phosphate dihydrate | 0.0565 | 40.2 |
| 4 | Colloidal silica | 0.0007 | 0.5 |
| 5 | HPMC | 0.0039 | 2.8 |
| 6 | Magnesium stearate | 0.0007 | 0.5 |
| Modified-release coating | | | |
| 7 | Neutral methacrylic co-polymer | 0.0032 | 2.3 |
| 8 | Talc | 0.0033 | 2.3 |
| 9 | Polysorbate 80 | 0.0005 | 0.4 |
| 10 | Hypromellose | 0.0008 | 0.6 |
| Color coating | | | |
| 11 | Hydroxypropyl methylcellulose, microcrystalline cellulose, stearic acid, dye | 0.0027 | 1.9 |
| | Total | 0.140 | 100 |

**Tablet B:**

| • Coated modified release tablet composition containi ng 14 mg zinc and 1 mg copper: | | | |
|---|---|---|---|
| No. | Material | Unit (g) | % |
| Active ingredients | | | |
| 1 | Zinc oxide | 0.0560 | 41.4 |
| 2 | D-gluconic acid copper (II) salt | 0.0071 | 5.2 |

| Excipients | | | |
|---|---|---|---|
| 3 | Microcrystalline cellulose | 0.0437 | 32.3 |
| 4 | Calcium hyd. phosphate dihydrate | 0.0219 | 16.2 |
| 5 | Colloidal silica | 0.0007 | 0.5 |
| 6 | Magnesium stearate | 0.0007 | 0.5 |

| Modified-release sealing coating | | | |
|---|---|---|---|
| 7 | Shellac | 0.0013 | 1.0 |

| Color coating | | | |
|---|---|---|---|
| 8 | Hydroxypropyl methylcellulose, TiO₂, hypromellose, glycerin, dye | 0.0039 | 2.9 |
| | Total | 0.135 | 100 |

**Tablet C:**

| Coated tablet composition containing 5 mg lutein and 1 mg zeaxanthin: | | | |
|---|---|---|---|
| No. | Material | Unit (g) | % |
| Active ingredients | | | |
| 1 | Lutein (20%) | 0.0250 | 18.7 |
| 2 | Zeaxanthin (10%) | 0.0100 | 7.5 |

| Excipients | | | |
|---|---|---|---|
| 3 | *Piper Nigrum* (95%) | 0.0002 | 0.1 |
| 4 | Microcrystalline cellulose | 0.0365 | 27.2 |
| 5 | Calcium hyd. phosphate dihydrate | 0.0371 | 27.7 |
| 6 | Sodium starch glycolate | 0.0039 | 2.9 |
| 7 | Colloidal silica | 0.0007 | 0.5 |
| 8 | Magnesium stearate | 0.0007 | 0.5 |

| Sealing coating | | | |
|---|---|---|---|
| 9 | Shellac | 0.0007 | 0.5 |

| Color coating | | | |
|---|---|---|---|
| 10 | Polyvinyl alcohol, TiO₂, Macrogol, talc, dye | 0.0192 | 14.3 |
| | Total | 0.134 | 100 |

## Claims

1. Encapsulated formulations comprising a capsule comprising tablets, microgranules, powder, or granulated material with some or all of the following components or food supplements:
a) plant extracts,
b) vitamins,
c) minerals,
d) amino acids.

2. Encapsulated formulations according to claim 1, **characterized in that** the capsule can be of plant or animal origin.

3. Encapsulated formulations according to any of claims 1 and 2, **characterized in that** the release of the tablets, microgranules, powder, or granulated material of a), b), c), and d) may or may not be modified.

4. Encapsulated formulations according to any of claims 1 to 3, comprising between 0 and 6 tablets with one or more of components a) to d) and microgranules, powder, or granulated material with the rest of the components.

5. Encapsulated formulations according to any of claims 1 to 3, comprising:
a) between 0 and 3 tablets with plant extracts,
b) between 0 and 3 tablets with vitamins,
c) between 0 and 3 tablets with minerals, and
d) between 0 and 3 tablets with amino acids.

6. Encapsulated formulations according to any of claims 1 to 5, **characterized in that** the plant extracts used are selected from: coenzyme Q10, *Hypericum*, *Curcuma kwangsiensis*, *Curcuma phaeocaulis, Curcuma zedoaria, Curcuma wenyujin, Curcuma aromatica, Echinacea* (*Equinacea angustifolia*) extract, *Harpagophytum procumbens*, *Harpagophytum zeyheri*, Mimosaceae (*Piptadeniastrum africanum*), *Passiflora* (*Passiflora caerulea*, *Passiflora edulis*, *Passiflora decaisneana*, *Passiflora manicata*) extract, *Valeriana* (*Valeriana collina*, *Valeriana pratensis*, *Valeriana repens*, *Valeriana sambucifolia*) extract, kava-kava (amount of extract equivalent to 100 mg of kavalactones), *Griffonia physocarpa*, *Griffonia speciosa*, *Griffonia tessmannii*, *Matricaria perforata*, *Matricaria recutita*, *Matricaria discoidea*, *Matricaria aurea*, *Tanacetum corymbosum*, *Tanacetum microphyllum*, *Chamaemelum nobile*, *Helichrysum italicum*, *Santolina chamaecyparissus*, saffron, L-5-hydroxytryptophan (5-HTP) extract, carotenoids (astaxanthin, lutein, Zeaxanthin), preferably 5-HTP, dry seed extract of *Griffonia simplicifolia*, *Tanacetum parthenium*, *Passiflora incarnata*, *Valeriana officinalis*, saffron, *Echinacea angustifolia*, astaxanthin, lutein, and zeaxanthin.

7. Encapsulated formulations according to any of claims 1 to 6, **characterized in that** the vitamins are selected from Vitamin A (retinol), B1 (thiamine), B2 (riboflavin), B3 (niacin), B5 (pantothenic acid), B6 (pyridoxine), B8 (biotin), B9 (folic acid), B12 (cobalamin), coenzyme Q10 (ubiquinone), C (ascorbic acid), D (calciferol), E (α-tocopherol), K (phylloquinone), preferably ascorbic acid coated with ethyl cellulose, pyridoxine hydrochloride, riboflavin, ubiquinone, calciferol, and vitamin K2.

8. Encapsulated formulations according to any of claims 1 to 7, **characterized in that** the minerals are selected from magnesium, calcium, phosphorus, sodium, potassium, chloride, iron, zinc, iodine, copper, manganese, fluorine, chromium, selenium, molybdenum, preferably magnesium oxide, iron, copper, zinc, and magnesium chloride.

9. Encapsulated formulations according to any of claims 1 to 8, **characterized in that** the amino acids or peptides are selected from tryptophan, valine, histidine, arginine, methionine, leucine, isoleucine, lysine, phenylalanine, threonine, glycine, proline, serine, asparagine, preferably eggshell membrane (OVOMET®), 90% marine chondroitin sulfate, vegetable D-glucosamine sulfate sodium (2NaCL), melatonin, and GABA (gamma aminobutyric acid).

10. Encapsulated formulations according to any of claims 1 to 9, **characterized in that** the tablets, microgranules, powder, or granulated material with any of components a) to d) can furthermore contain excipients of the type: diluent, binder, disintegrant, lubricant, surfactant, sweetener, dye, preservative, stabilizer, flavor enhancer, pH regulator, coatings.

11. Encapsulated formulations according to any of claims 1 to 10, **characterized in that** the diluent comprises hydroxypropyl methylcellulose HPMC, calcium phosphate, lactose, dextrose, sugar, starch, modified starch, corn starch, mannitol, sorbitol, inorganic salts, microcrystalline cellulose, cellulose, calcium sulfate, xylitol, and/or lactitol.

12. Encapsulated formulations according to any of claims 1 to 11, **characterized in that** the tablets and/or microgranules can be formed by an inert lipophilic or hydrophilic matrix which modifies the solubilization of the different ingredients making up same to provide different release characteristics.

13. Encapsulated formulations according to any of claims 1 to 12, **characterized in that** the tablets and/or microgranules can be coated with a protective coating, enteric coating, dye, and/or film to provide different release characteristics.

14. Use of a capsule according to any of claims 1 to 13 for producing a food supplement for the prevention and treatment of different types of disorders.

15. Use of a capsule according to any of claims 1 to 14 for producing a food supplement for the prevention and treatment of sleep disorders.

16. Use of a capsule according to any of claims 1 to 14 for producing a food supplement for the prevention and treatment of anxiety.

17. Use of a capsule according to any of claims 1 to 14 for producing a food supplement for the prevention and treatment of joint pain.

18. Use of a capsule according to any of claims 1 to 14 for producing a food supplement for the prevention and treatment of migraines.

19. Use of a capsule according to any of claims 1 to 14 for producing a food supplement to aid in the normal functioning of the immune system.

20. Use of a capsule according to any of claims 1 to 14 for producing a food supplement to aid in the normal functioning of the eye.
